# EUROPEAN PATENT APPLICATION

(11) **EP 2 687 224 A1**
(43) Date of publication of application: **22.01.2014**
(21) Application number: 12177127.3
(22) Date of filing: 19.07.2012
(51) Int. Cl.: A61K 38/17

(54) **Medicament for wound treatment**

(71) Applicant: Tissue Med Biosciences Forschungs- und Entwicklungsgesellschaft mbH, 3500 Krems (AT)
(72) Inventor: Berger, Rudolf, 3500 Krems (AT)
(74) Representative: Sonn & Partner Patentanwälte

(57) **Abstract**

The invention relates to stathmin, stathmin, a nucleic acid encoding said stathmin or a cell expressing stathmin for use in the treatment of chronic wounds or disease- or medication-dependent impaired wound healing in a patient or for increasing or inducing the proliferation and/or migration of mesenchymal cells, of stem cells, for stimulating immune cells, preferably natural killer cells, for stimulating fibroblasts, for stimulating epithelial cells, preferably epithelial cells of the epidermis, or for stimulating angiogenesis.

## Description

### Field of the invention

The present invention relates to the field of medicaments for regenerative treatments, in particular wound healing.

### Background

Wound healing constitutes a complex, dynamic, and well-orchestrated process that is activated whenever disruption of the skin tissue occurs. A wide spectrum of events takes place through the wound-healing process including platelet aggregation, coagulation cascade activation, cell migration, cell proliferation, inflammatory infiltration, cellular differentiation, and tissue remodeling. Although the cascade of these events seems to be well discriminated and divided into three phases, namely inflammation, proliferation, and wound contraction and remodeling, they actually overlap with each other and continued or renewed tissue damage may re-initiate the sequence at affected sites within an existing wound.

Skin tissue injury and disruption of blood vessels is followed by the formation of the fibrin clot, which provides the substrate for cell migration into the wound. Neutrophils, attracted by a range of molecules, phagocytose foreign bodies and bacteria being gradually replaced by monocytes that differentiate, to macrophages. Macrophages phagocytose microorganisms, fragments of the extracellular matrix, fibrin, neutrophils, erythrocytes, and other debris and secrete a variety of molecules that are considered crucial for effective wound repair. In chronic wounds, one or more of the components of this phase seem to be dysregulated, leading to delayed wound healing. Growth factor expression from keratinocytes, fibroblasts, and smooth muscle cells is diminished in the diabetic wound, negatively affecting the formation of the initial hemostatic plug. In addition, a hyperglycemia- as well as hyperlipidemia - related impairment of cytokine release, chemotaxis, adherence, and phagocytic activity have been observed.

Impaired wound healing is associated with increased morbidity and mortality. Non-healing wounds often lead to amputation. However, despite an extensive research, the exact underlying pathogenetic mechanisms have not been fully elucidated. It appears to be the net result of micro- and macrovascular disease. Wound healing is typically delayed in diabetes mellitus and angiopathy seems to contribute significantly. In addition, neuropathy and the lack of growth factors supporting mesenchymal cell migration and activation has been recognized as one of the causes for delayed diabetic wound healing. Other factors that appear to be innate to the diabetic state itself, such as changes in cell morphology and function, have been implicated in its pathogenesis.

WO 2007/089151 A1 identified stathmin as TLR3 activating substance and suggested the treatment of degenerative inflammatory processes in neurodegenerative disorders such as Alzheimer's disease. Also the treatment of wounds is mentioned.

On the other hand US 6,429,304 B1 teaches compounds with anti-mitotic activity that can be used to treat various proliferative diseases such as cancer or "abnormal wound healing". The mode of action is a prevention of cell growth. The anti-mitotic activity of the compounds is based on an alleged microtubuli depolymerization ability. One such compound is stathmin, also referred to as OP18.

WO 2004/113561 A2 is a further document stating that stathmin or oncoprotein 18 prevents uncontrolled cell growth due to tubulin decreases.

None of these documents have investigated the role of stathmin in wound healing in practice. The authors of these patent documents further mentioned opposing statements on the possible effect in wound healing. It thus remains to be elucidated if stathmin has any beneficial effect in wound healing.

Current wound healing treatments aim at cleaning and debriding the wound, treating possible infections to reduce the inflammatory phase of the wound, and optionally applying migration or growth factors such as PDGF. Still, current wound treatment formulations are unsatisfying and there is an ongoing need for improved wound healing compositions.

### Summary of the Invention

The present invention provides the use of stathmin, a nucleic acid encoding stathmin or a cell expressing stathmin in the treatment of wounds. In a related aspect, the present invention also relates to stathmin, a nucleic acid encoding stathmin or a cell expressing stathmin for use in the treatment of wounds. Further, the invention relates to the use of stathmin, a nucleic acid encoding stathmin or a cell expressing stathmin in the preparation of a medicament in the treatment of wounds. Also provided are pharmaceutical compositions comprising stathmin. The invention further provides the *in vitro* or *in vivo* method of increasing or inducing the proliferation and/or migration of mesenchymal cells, epidermal cells and/or stem cells, in particular stromal stem cells, for stimulating immune cells, preferably natural killer cells, for stimulating fibroblasts, for stimulating epithelial cells, preferably epithelial cells of the epidermis, or for stimulating angiogenesis, in particular for stimulating IL-8 production in said cells, especially in mesenchymal cells and/or fibroblasts. All of these aspects are equivalent and all preferred embodiments relate to each one of these aspects equally.

The present inventors have identified stathmin as a very potent wound healing accelerator, that even surpassed common medicaments such as PDGF formulations. In particular surprising was the effect in case of reduced or impaired wound healing, where stathmin could achieve wound closure at an unexpected fast rate. The subject matter of the present invention is further defined in the claims.

### Detailed Description

Stathmin is a protein with known sequence (NCBI database NP_005554 and CAD19057 or SEQ ID NO: 1 (stathmin a) or SEQ ID NO: 2 (stathmin b)). Stathmin and its isoforms are further known from the background art mentioned above. It is an ubiquitously expressed protein found in most tissues in mammals. Many different phosphorylated forms are observed depending on specific combinations among the sites which can be phosphorylated. Phosphorylation at Ser-16 seems to be required for neuron polarization. Phosphorylation at Ser-63 reduces tubulin binding 10-fold and suppresses the MT polymerization inhibition activity.

SEQ ID NO 1:
MASSDIQVKE LEKRASGQAF ELILSPRSKE SVPEFPLSPP KKKDLSLEEI QKKLEAAEER 60
RKSHEAEVLK QLAEKREHEK EVLQKAIEEN NNFSKMAEEK LTHKMEANKE NREAQMAAKL 120
ERLREKDKHI EEVRKNKESK DPADETEAD 149

SEQ ID NO 2:
MASSDIQVKE LEKRASGQAF ELILSPRSKE SVPEFPLSPP KKKDLSLEEI QKKLEAAEER 60

RKSHEAEVLK QLAEKREHEK EVLQKAIEEN NNFSKMAEEK LTHKMEANKE NREAQMAAKL 120
ERLREKMYFW THGPGAHPAQ ISAEQSCLHS VPALCPALGL QSALITWSDL SHHH 174

Secreted Stathmin was identified by the inventors in a human cell line resembling plasmacytoid dendritic cells (pDC). Plasmacytoid dendritic cells (pDCs) have a distinctive role in wounded skin and are involved in the acute inflammatory response and wound healing through their production of growth factors. A human dendritic cell line was developed which secretes a unique set of proteins, which are instrumental for tissue regeneration. One of these proteins is stathmin, a protein with a molecular weight of 17,3kD (SEQ ID NO: 1). The stathmin gene from pDCs was cloned and expressed in E. coli and purified to homogeneity.

*In vitro,* stathmin induces proliferation and migration of human mesenchymal cells, supports neoangiogenesis and stimulates natural killer cell activity. The absence of dermal cell proliferation, new vessels and a first line defense against bacterial infections are the key problems in many chronic wounds. Striking results were obtained *in vivo* when wound healing in *Zucker* rats which have a delayed wound healing due to a metabolic syndrome was studied. When topically applied in a hydrogel, stathmin significantly outperformed PDGF. Moreover, PE-tomography with ¹²⁴I-labelled stathmin showed that the protein remained in the wounded area. Stathmin is remarkable stable in wound fluid (more than 8 hrs) and is not detectable in serum when applied topically, no systemic exposure was measured.

Based on these new in vitro and in vivo results, the present invention provides for the first time a use of stathmin in the treatment of wounds supported by experimental evidence.

In a central aspect, the present invention further provides the use of stathmin in non-healing wounds (non-healing without the inventive treatment) or in treating wounds with reduced or impaired wound healing. Wounds with reduced or impaired wound healing are e.g. chronic wounds or wounds with disease- or medication-dependent impaired wound healing (which may also be or develop into chronic wounds). Likewise it is also possible to use nucleic acids that encode stathmin and lead to expression thereof in a cell or the use of such a cell in such a treatment.

The invention further provides the combination of stathmin with collagen in the treatment of wounds. This combination shows even further increased wound healing rates due to synergistic effects.

Stathmin, as used herein relates to any stathmin isoform or variant or any post-translationally modified form, including phosphorylated forms. In preferred embodiments stathmin comprises amino acids 1 to 126 of SEQ ID NO: 1, or an amino acid sequence that has at least 70 % sequence identity with the sequence of amino acids 1 to 126 of SEQ ID NO: 1. The amino acids 1 to 126 of SEQ ID NO: 1 are also found in SEQ ID NO: 2 and appear to be of a homologous region. Further preferred stathmin variants are disclosed in WO 2007/089151, US 6,429,304 B1, WO 2004/113561 A2 and NCBI database entries NP_005554 and CAD19057 (all incorporated herein by reference).

The term "sequence identity" refers to identity between two sequences, usually amino acid sequences, which can be determined by sequence alignment programs like BLAST, PSI-BLAST (www.ncbi.nlm.nih.gov/blast/) or ClustalW (www.ebi.ac.uk/clustalw/). These algorithms calculate the best match for the selected sequences, and line them up so that the identities, similarities and differences can be seen.

In preferred embodiments of the present invention the inventive stathmin comprises a sequence with at least 75 %, more preferred at least 80 %, at least 85%, at least 90%, at least 95%, at least 98% or even 100%, sequence identity with the sequence of amino acids 1 to 126 of SEQ ID NO: 1. In even more preferred embodiments the inventive stathmin comprises a sequence with at least 70 %, more preferred at least 75%, at least 80 %, at least 85%, at least 90%, at least 95%, at least 98% or even 100%, sequence identity with the sequence as set forth in SEQ ID NO: 1. Stathmin may be stathmin-1, stathmin-2, stathmin-3 or stathmin-4, preferably it is stathmin-1. Stathmin can be a recombinant stathmin. Further preferred it is of the same origin as the patient but also stathmin variants from other animals can be used. Preferably the patient is a mammal, especially a human or non-human animal, in particular a domestic animal, such as pig, rodents, or a primate. Preferably the stathmin is human stathmin or stathmin from a non-human animal, in particular a domestic animal, such as pig, rodents, or a primate.

As used herein "comprising" is used in an open meaning, i.e. that the stathmin of the present invention may have further amino acids or protein components. It may be a fusion protein. Such extended stathmin proteins may have in preferred embodiments a limited size, e.g. up to 2000 amino acids, up to 1800 amino acids, up to 1600 amino acids, up to 1400 amino acids, up to 1200 amino acids, up to 1000 amino acids, up to 800 amino acids, up to 600 amino acids, up to 400 amino acids, up to 300 amino acids, up to 200 amino acids, or up to 160 amino acids. Of course the invention also relates to stathmin proteins that consist of any one of said sequences comprised in the above mentioned embodiments. "Consisting" is used in a closed and sequence limiting meaning.

In preferred embodiments stathmin is phosphorylated. Phosphorylation is a natural post-transcriptional process and occurs during recombinant expression. Possible phosphorylation sites are at amino acids corresponding to amino acids 16, 25, 28, 38, 63, 146 of SEQ ID NO: 1. Preferred phosphorylation are at amino acids corresponding to amino acids 16 and/or 63 of SEQ ID NO: 1. The inventive stathmin may comprise 1, 2, 3, 4, 5 or 6 amino acid phosphorylations.

In preferred embodiments stathmin is acetylated. Acetylation is a natural post-transcriptional process and occurs during recombinant expression. Possible acetylation sites are at amino acids corresponding to amino acids 2, 9, 80, 95, 100, 119, 128 of SEQ ID NO: 1. The inventive stathmin may comprise 1, 2, 3, 4, 5, 6 or 7 amino acid acetylations.

The stathmin can be glycosylated or not glycosylated. E. coli produced stathmin is not glycosylated and still effective.

Alternatively (or in combination) to using stathmin proteins it is also possible to use nucleic acids that encode the above mentioned stathmin protein. Such a sequence is e.g. set forth in SEQ ID NO: 3, which encodes SEQ ID NO: 1.

SEQ ID NO 3:
atggcttctt ctgatatcca ggtgaaagaa ctggagaagc gtgcctcagg ccaggctttt 60
gagctgattc tcagccctcg gtcaaaagaa tctgttccag aattccccct ttcccctcca 120
aagaagaagg atctttccct ggaggaaatt cagaagaaat tagaagctgc agaagaaaga 180
cgcaagtccc atgaagctga ggtcttgaag cagctggctg agaaacgaga gcacgagaaa 240
gaagtgcttc agaaggcaat agaagagaac aacaacttca gtaaaatggc agaagagaaa 300
ctgacccaca aaatggaagc taataaagag aaccgagagg cacaaatggc tgccaaactg 360
gaacgtttgc gagagaagga taagcacatt gaagaagtgc ggaagaacaa agaatccaaa 420
gaccctgctg acgagactga agctgactaa 450

Nucleic acids can be used to induce production of stathmin in cells near the wound. Preferred formulations for nucleic acid delivery are e.g. liposomes, microemulsions, micelles or vesicles for controlled delivery. The cell may then produce and secrete stathmin to provide a continuous production of the therapeutic agent.

Also provided is a cell that expresses stathmin for the inventive uses. Such a cell preferably continuously secretes stathmin to provide for the therapeutic effect. Such a cell can be any cell. Preferably the cell is a pDC but also non-pDC's are possible. In preferred embodiments the cell is not-immunogenic to the patient, e.g. it is a cell obtained from the patient that has been genetically engineered to recombinantly express stathmin. This modification of the cell can be performed in vitro or in vivo.

The previous and further detailed description relates to the stathmin protein, the nucleic acid and the cells equally, in particular since the directly active therapeutic agent of the nucleic acid or the cell is also the expressed stathmin.

The present invention provides in particular stathmin, a nucleic acid encoding said stathmin or a cell expressing stathmin for use in the treatment of chronic wounds or wounds with defective angiogenesis in a patient. Preferably the treated tissue is the skin or the treated tissue or area comprises at least in part the skin. Chronic wounds or wounds with impaired wound healing may also affect subcutaneous tissues, even the bone. The treatment is preferably topical, especially with a topical formulation such as a hydrogel.

Stathmin, the nucleic acid or the cell can be formulated with or coupled to a biocompatible matrix, preferably comprising collagen, gelatin, chitosan and/or hyaluronan, for use in the treatment of wounds, preferably chronic wounds or wounds with defective angiogenesis, in a patient. In fact, this formulation provides surprisingly new properties and effects that are novel over any other prior formulation. Therefore the invention also provides the use of stathmin in such a formulation for the treatment of wounds, including acute and chronic wounds and wounds with impaired wound healing. The preparation of biocompatible matrices is well known in the art and e.g. described in Tan et al. (9). Biocompatible matrices preferably support wound healing and/or adhesion of cells, in particular in the wound. They lead to a strengthening of the regrown cellular scar tissue. Preferably the biocompatible matrix comprises a scaffold with hyaluronic acid.

In preferred embodiments the stathmin is formulated in a hydrogel. Hydrogels preferably comprise gelatin, alginate, agarose, methylcellulose, hyaluronan or any combination thereof. Organo-chemical hydrogels may comprise polyvinyl alcohol, sodium polyacrylate, acrylate polymers and copolymers with an abundance of hydrophilic groups. Hydrogels comprise a network of polymer chains that are hydrophilic, sometimes found as a colloidal gel in which water is the dispersion medium. Hydrogels are highly absorbent (they can contain over 99.9% water) natural or synthetic polymers. Hydrogels also possess a degree of flexibility very similar to natural tissue, due to their significant water content. They provide a reservoir in topical drug delivery.

In preferred embodiments the wound to be treated is a chronic wound. A chronic wound shows no or limited wound healing. It is a wound that does not heal in an orderly set of stages and in a predictable amount of time. In particular embodiments, the chronic wound is a wound that has no or reduced wound healing (e.g. only about 20 % wound area closure) within 14 days, preferably 18 days, especially preferred 22 days, even more preferred 28 days, within 34 days, 40 days, 50 days or even 60 days. In preferred embodiments the wound is anoxic and/or lacks sufficient oxygen supply and/or lacks newly formed arteries. The present invention also stimulates angiogenesis and can help to restore proper or even accelerated wound healing as well as angiogenesis, in a wound or elsewhere.

Alternatively or in combination, the wound is a wound with disease-dependent impaired wound healing (disease-dependent impaired wound healing can also be chronic as defined above). Disease-dependent impaired wound healing can due to a disease selected from diabetes or metabolic syndrome, chronic venous insufficiency (CVI), peripheral artery occlusive disease (PAOD), cancer, autoimmunity, especially an autoimmunity selected from rheumatoid arthritis, lupus (in particular systemic lupus erytematodes) and livedoid vasculopathy, surgical wounds, ostomy, prolonged inflammatory processes, cellular senescence such as in decubitus growth factor deficiency or growth factor receptor deficiency. The patient treated according to the invention may have one or more of these diseases or conditions. Preferably said patient suffers from diabetes or metabolic syndrome.

Alternatively or in combination, the wound is a wound with medication-dependent impaired wound healing. Medication-dependent impaired wound healing can be due to a medication selected from treatments with a cortiocosteroid, nicotine, an antibiotic, an immunosuppressant, an anti-coagulant, a cytotoxic medication, an anti-rheuma-medication, especially an anti-rheumatoid arthritis medication, a vasoconstrictor. The patient treated according to the invention may have received one or more of these medications.

Thus the present invention also provides for the use of stathmin for stimulating angiogenesis. A patient with tissues with insufficient artery supply may be treated. The patient may have anoxic tissues that are treated by the present invention. The tissue with insufficient artery supply may also be chronic, e.g. be in this state for 14 days, preferably 18 days, especially preferred 22 days, even more preferred 28 days, for 34 days, 40 days, 50 days or even 60 days.

Chronic diseases may have acute causes, such as surgical or accidental wounds, or chronic causes, such as in diabetes or other comorbidities, especially a combination with other diseases that cause reduced oxygenation of the skin or other tissues.

Thus, the present invention relates to a treatment of a patient who is in need of an induction of angiogenesis with stathmin, in particular in the tissue in need of such therapy. Preferably the therapy is topical (as is preferred for all embodiments of the invention).

In preferred embodiments the patient does not suffer from a systemic degenerative inflammatory process. The treated wound may or may not comprise a degenerative inflammatory process.

Further treatments are of patients who are in need of a stimulation of the innate immune response and/or in need of a induction of cytokines/growth factors and/or TIMPs for immune modulation. Such patients may have a locally or systemic immune deficiency. The immune deficiency may be an insufficient supply (locally or systemically) of all immune cells or a limited immune cell activation or a limited supply (locally or systemically) of particular immune cells, especially cells of the innate immune system, such as NK cells, which are preferably stimulated according to the invention. Locally preferably relates to the wounded area. Preferably the immune deficiency is a deficiency in cell-mediated immunity and/or deficiency in the innate immune system. The immune system is divided into a more primitive innate immune system, and acquired or adaptive immune system, each of which contains humoral and cellular components.

Stathmin, a nucleic acid or cell can be used to treat a wound in a patient who suffers from diabetes or metabolic syndrome. Diabetes and the metabolic syndrome cause a dysregulation in the patients proliferative and metabolic capabilities, which leads to reduced or no wound healing. Further, diabetes causes immune compromise and damage to small blood vessels, preventing adequate oxygenation of tissue, which can cause chronic wounds even without acute causes.

Particular examples of wounds or conditions that can be treated with stathmin are wounds selected from diabetic foot wound, especially a diabetic foot ulcer, an ulcer in general, in particular venous ulcer, a decubitus or pressure ulcer, burns, preferably a third degree burn, a surgical wound, an accidental wound, a necrotic wound, an infected wound. These wounds and conditions may be chronic but in special cases also acute. In preferred embodiments, chronic versions of all of these wounds and conditions are treated according to the invention.

The present invention further relates to a pharmaceutical composition comprising stathmin. Pharmaceutical compositions or formulations for therapeutic use may comprise a pharmaceutically acceptable diluent, carrier, solubilizer, emulsifier, preservative and/or adjuvant. The invention also provides for pharmaceutical compositions comprising a therapeutically effective amount of stathmin. The term "therapeutically effective amount" means an amount which provides a therapeutic effect for a specified condition and route of administration. The composition may be in a liquid or lyophilized form and comprises a diluent (Tris, acetate or phosphate buffers, NaCl) having various pH values and ionic strengths, solubilizer such as Tween or Polysorbate, carriers such as human serum albumin or gelatin, preservatives such as thimerosal or benzyl alcohol. Selection of a particular composition will depend upon a number of factors, including the condition being treated, the route of administration and the pharmacokinetic parameters desired.

Preferred formulations are formulations for topical administration. Especially preferred is a hydrogel. Also encompassed are compositions comprising stathmin modified with water soluble polymers to increase solubility, stability, plasma half-life and bioavailability. Compositions may also comprise incorporation of stathmin into liposomes, microemulsions, micelles, microparticles or vesicles for controlled delivery over an extended period of time.

In special embodiments stathmin is provided with a carrier. The carrier is preferably selected from a gel, preferably a hydrogel, or a wound dressing or a swab, optionally impregnated with a solution containing stathmin. Further carriers comprise carriers for slow-release which release the active agent combination as a longer effective application delayed or slower. Such a preparation with a corresponding carrier is especially suitable for topical and quick administration.

In preferred embodiments, stathmin is used in amounts of between 0.1 µg to 1000 µg stathmin per cm² of the wound. Also preferred are amounts of at least 0.1 µg at least 0.2 µg, at least 0.5 µg, at least 1 µg, at least 2 µg, at least 5 µg and/or at most 1000 µg, at most 750 µg, at most 500 µg, at most 400 µg, at most 300 µg, at most 200 µg, at most 100 µg or at most 50 µg per cm² of the wound or any range in between these values.

The pharmaceutical composition may comprise stathmin formulated with or coupled to a biocompatible matrix, preferably comprising collagen, gelatin, chitosan and/or hyaluronan, as described above. The pharmaceutical composition may also comprise a hydrogel with stathmin as described above.

In particular, the present invention provides the pharmaceutical composition for use as a medicament. Particular uses are in wound healing and/or the stimulation of angiogenesis as described above.

In a further aspect the present invention also provides a method of increasing or inducing the proliferation and/or migration of mesenchymal cells, epidermal cells and/or stem cells, in particular stromal stem cells or hematopoietic stem cells, for stimulating immune cells, for stimulating fibroblasts, for stimulating epithelial cells, preferably epithelial cells of the epidermis or of blood vessels, or for stimulating angiogenesis. This method can be performed *in vitro,* e.g. on isolated cells or cell cultures. In particular the inventive method can be used for stimulating IL-8 production in said cells, especially in mesenchymal cells and/or fibroblasts. The invention stimulates IL-8 production, which has particular beneficial effects on wound healing.

Also provided is the use of this method in vivo, in particular on a patient with a wound or in need of such a treatment, e.g. for stimulating angiogenesis. Thus provided is the method of increasing or inducing the proliferation and/or migration of mesenchymal cells, epidermal cells and/or stem cells, in particular stromal stem cells or hematopoietic stem cells, for stimulating immune cells, for stimulating fibroblasts, for stimulating epithelial cells, preferably epithelial cells of the epidermis or of blood vessels, or for stimulating angiogenesis in a patient comprising the step of administering stathmin, a nucleic acid encoding said stathmin or a cell expressing stathmin to the patient, preferably by topical administration on a wound. The patient may be in need of such increase, induction or stimulation, e.g. due to a wound, or anoxic, necrotic or infected (or a combination thereof) tissue. In particular the inventive method can be used for stimulating IL-8 production in said cells, especially in mesenchymal cells and/or fibroblasts as stated above.

Immune cells that can be stimulated are preferably cells of the innate immune system, in particular natural killer cells.

The present invention further provides the stimulation of any one of the growth factors as given in table 2 below, especially IFN-alpha, IFN-beta, PDGF-B, FGF-2, HGF, TGF-beta, VEGF and/or KGF, or of IL-8. This method can be used in the above described in vitro or in vivo method.

The present invention will be further explained by the following figures and examples without being limited to theses specific aspects of the invention.

### Abbreviations

DFU, diabetic foot ulcer
FCS, fetal calf serum
FN, fibronectin
FPLC, fast protein liquid chromatography
HIC, hydrophobic interaction chromatography
Hrs, hours
ON, overnight
pDC, plasmacytoid dendritic cell
PDGF, platelate derived growth factor
R hu Stat1, recombinant human Stathmin1
TIMP, tissue inhibitor of metalloproteinases
TLR, toll-like receptor

### Figures

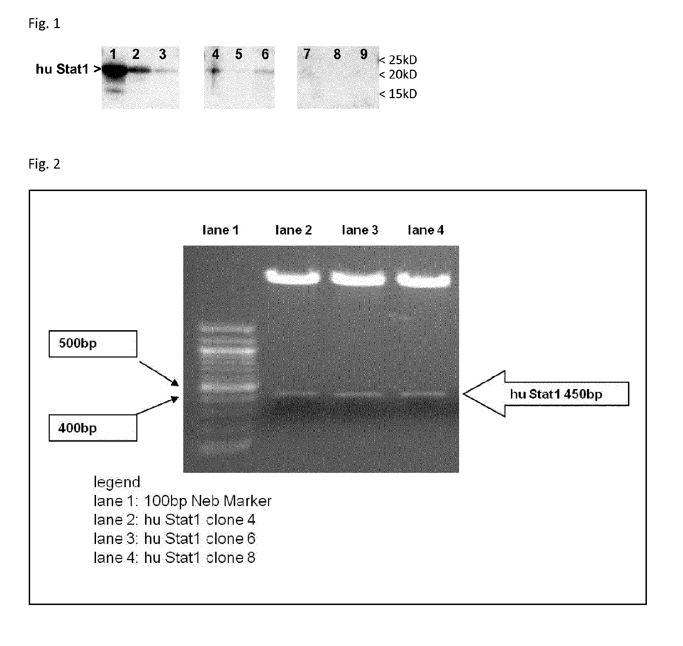

Fig.1: Secretion of hu Stat1 by human pDC-like cells. AB4 (human pDC) and PS cells (primary human skin fibroblasts) were cultured in serum-free medium for 48hrs. Culture supernatants or cell lysates were harvested and analyzed by western blotting using a rabbit anti-hu Stat1 antiserum. Lanes 1-3: recombinant hu Statlas a positive control (500ng, 50ng and 5 ng), lanes 4-6: 4) AB4 lysate 4x10⁴ cells, AB4 SN 10⁴ cells, AB4 SN 4x10⁴ cells; lanes 7-9: PS lysate 4x10⁴ cells, PS SN 10⁴ cells, PS SN 4x10⁴ cells.
Fig.2: Cloning of hu Stat1 from AB4 cells. Plasmid DNA of hu STAT1 in pTriEx4 Neo/TOP10 clone 4 was transformed into BL21 DE 3 chemically competent cells with heat shock at 42°C for 30 seconds. Transformation reaction was plated out on LB Agar plates with Ampicillin and incubated for 12 hours at 37°C. Colony PCR and control digest were carried out as described above. After NcoI/Bsu36I control digestion hu STAT1 in pTriEx4 Neo BL21 DE3 clones showed the respective band at 450bp in an agarose gel.

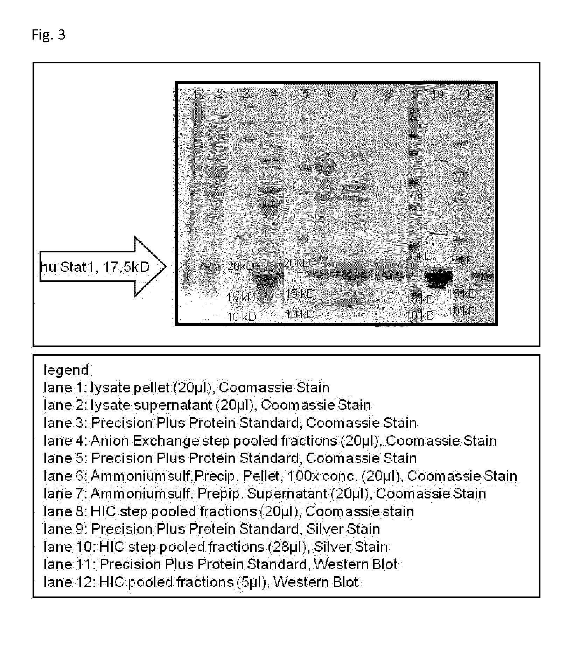

Fig.3: Purification of hu Stat1 by sequential anion exchange and hydrophobic interaction chromatography. An ÄKTA Purifier FPLC System was used for DEAE Sepharose and Octyl Sepharose Fast Flow chromatography.

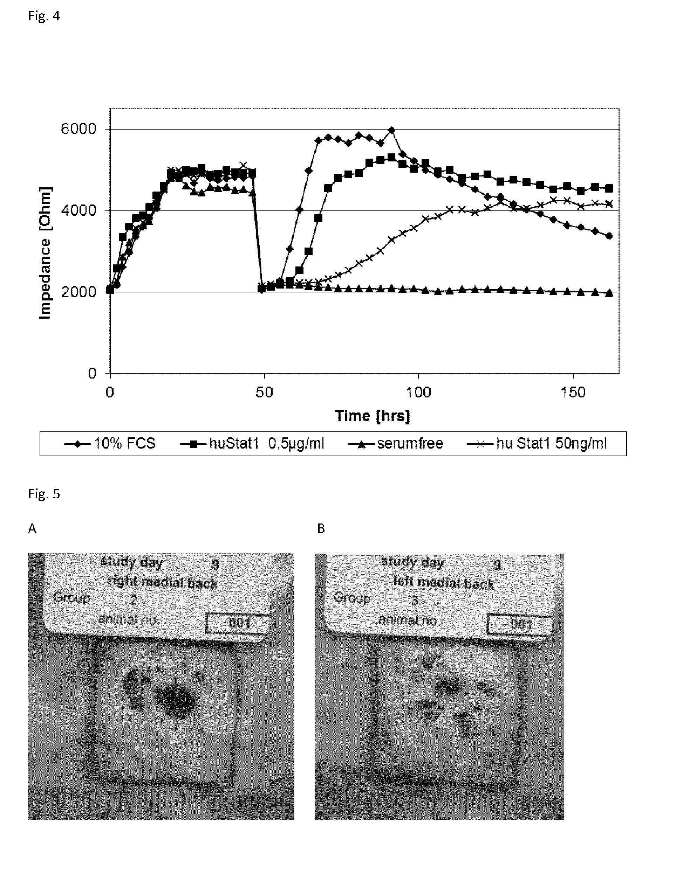

Fig.4: Electric cell-substrate impedance sensing (ECIS) test - hu Stat1 mediated in vitro wound healing. Human epithelial cells (A431) were cultured in fibronectin-coated culture vessels (8W1E, IBIDI, Munich). After 48hrs a high-field pulse was employed (see arrow) which resulted in a sharp drop of impedance and cell death at the area covered by the electrode. Addition of hu Stat1 lead to proliferation and migration of cells into the wounded area in a dose dependent manner. 10% FCS: pos. control, serum-free medium: neg. control
Fig.5: Treatment of Zucker rats with collagen alone (A) or a combination of stathmin with collagen (B). Shown are photographs on day 9 after treatment start of differently treated wounded spots on the same rat.

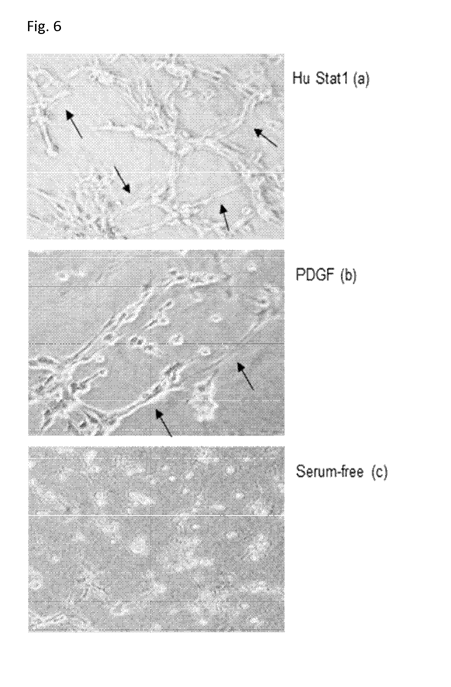

Fig.6 : Induction of tube-like structures and lumina by Stat1. Human endothelial cells (ECV304) were grown on fibronectin-coated culture vessels in serum free medium. After addition of recomb. Hu Stat1 (0.5ng/ml) or PDGF (1ng/ml), cultures were analyzed by transmission light microscopy (x200) after 3 days. Stat1 treated cultures clearly showed sprouting (see arrowheads) while this phenomenon was essentially absent in serum-free cultures.

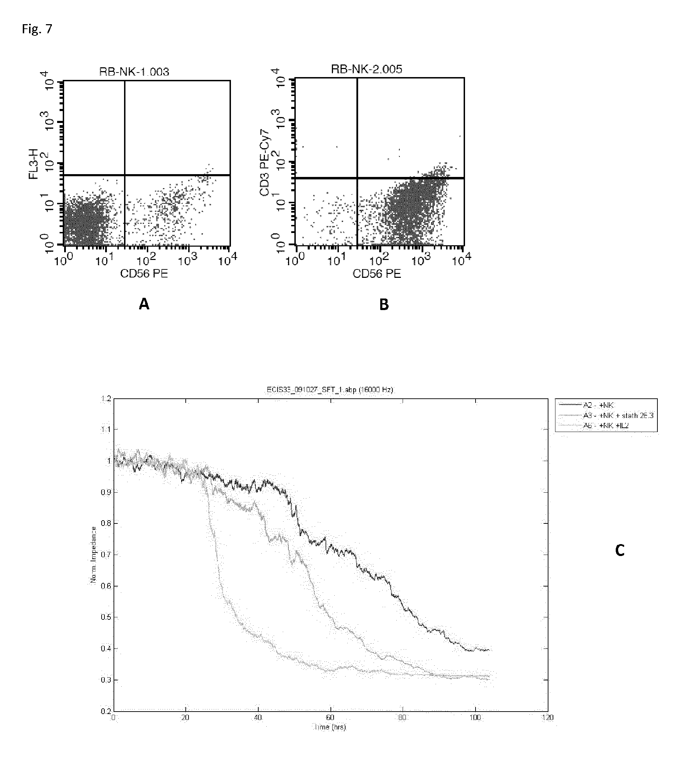

Fig. 7: Recombinant hu Stat1 stimulates activity of human NK cells. Human NK cells were isolated from human peripheral blood lymphocytes by MACS Separation (Miltenyi Biotec, #130-092-657) resulting in a population of <97% CD56+ NK cells(B). The percentage of CD56+ cells pre-isolation was 13% (A). CD56+ - enriched cells served as effectors in a cytotoxicity assay using A431 cells as targets (E:T ratio: 10:1) and the ECIS-test system as a read- out system (C). The blue line indicates the activity of NK cells in the presence of hu IL-2 (50U/ml) and the green line NK cells plus hu Stat1 (0.5µg/ml). NK cells in the presence of medium only (red, top line) never reached the activity of Stat1 or IL-2 treated cells.

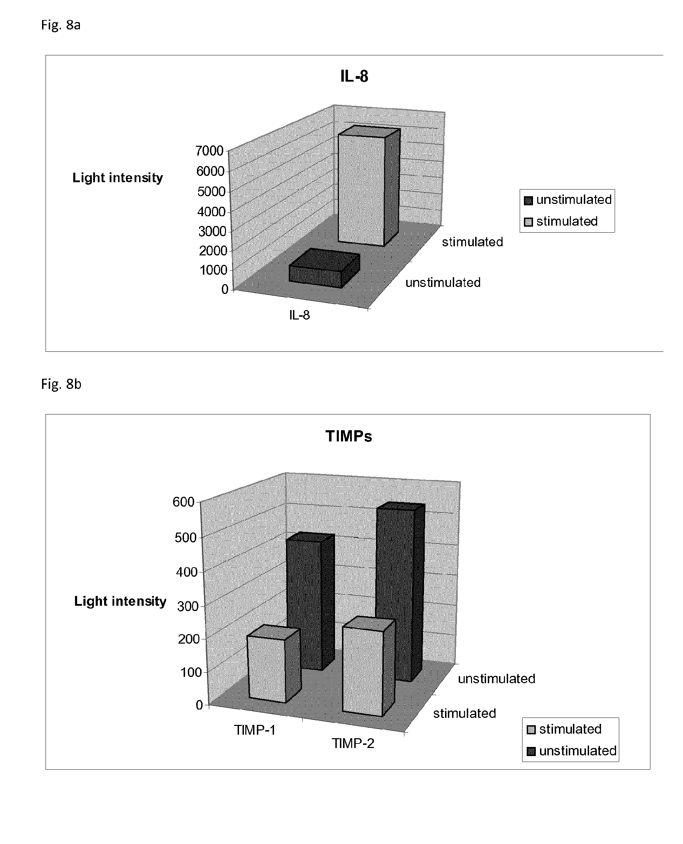

Fig.8a: Induction of hu IL-8 by recombinant Stathmin1 in human primary skin fibroblasts. Human primary skin fibroblasts were cultured in serum-free medium without (red bar) or in the presence of 50 ng hu Stat1 (green bar). Cells were harvested after 18 hours, lysed and analyzed by a protein array (R&D) specific for soluble receptor molecules. Protein arrays were analyzed by chemoluminescence.
Fig.8b: Downregulation of TIMP-1 and -2 by recombinant Stathmin1 in human primary skin fibroblasts. Human primary skin fibroblasts were cultured in serum-free medium without (red bar) or in the presence of 50 ng hu Stat1 (green bar). Cells were analyzed as described in Fig.8a.

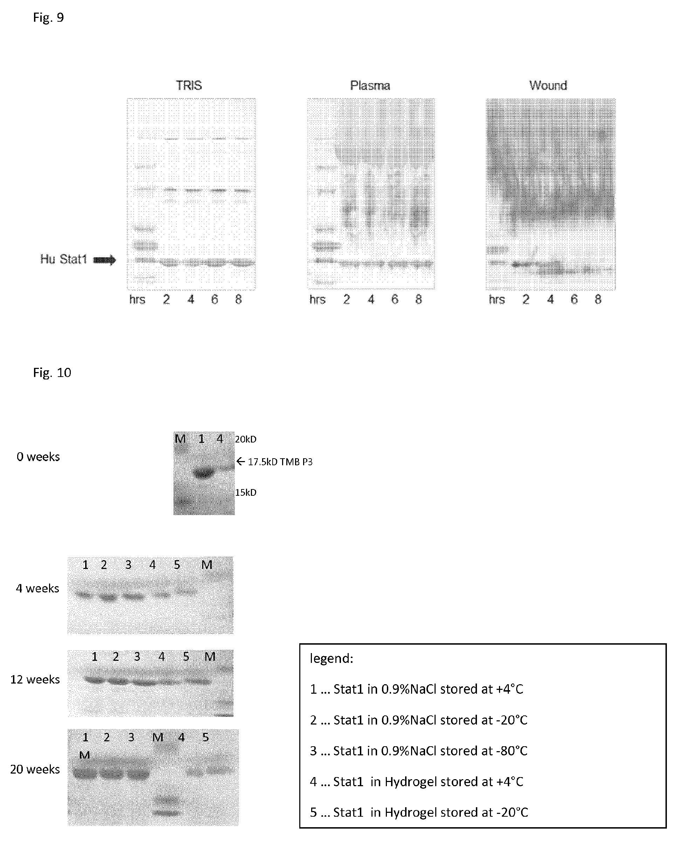

Fig.9: Stability of hu Stat1 in wound fluid at 37°C. 1.5 µg hu recombinant Sta1 was mixed with either Tris-buffer, human plasma or wound fluid derived from a chronic wound, incubated at 37°C for the time indicated, separated by SDS-PAGE and further analyzed by western blotting using a rabbit anti hu Stat1 serum.
Fig.10: Hu Stat1 is stable for at least 5 months in in hydrogel. Hu recombinant Stat1 was dissolved in either 0.9%NaCL or mixed in a hydrogel and stored at the temperatures indicated. 1 µg Stat1 was analyzed after 1, 3 and 5 month by SDS-PAGE and Co-massie staining.

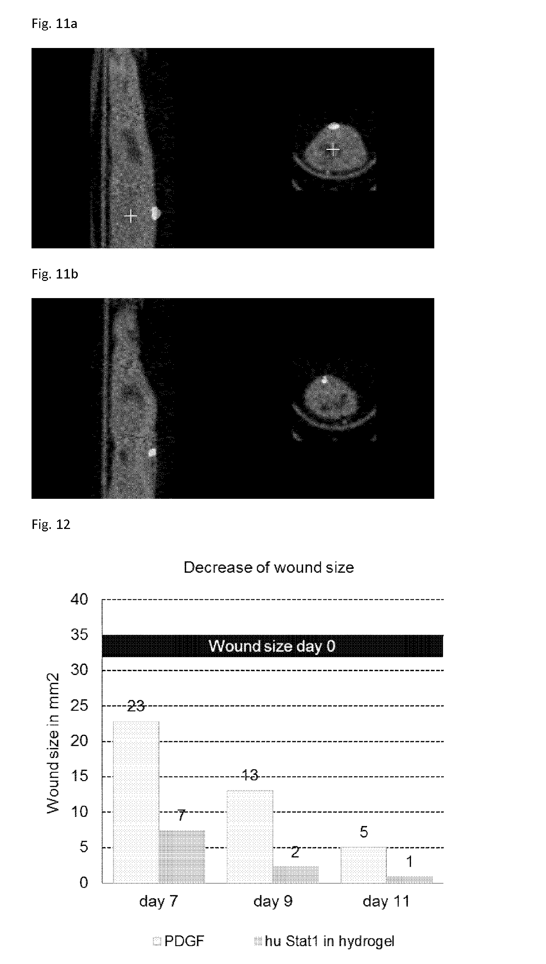

Figure 11a: Micro-PET Imaging. Projection image of the summed data (3 bed positions, each 10-min scan) from one rat after administration of 124I-hu Stat1 (5 µg). The wound can be identified in the image by a sharp white spot.
Figure 11b: Micro-PET Imaging. Projection image of the summed data (3 bed positions, each 10-min scan) from one rat 24 hrs after administration of 124I-hu STAT1 (5 µg). The wound can be identified in the image by a sharp white spot.
Figure 12: Efficacy of hu Stat1 in a wound healing model in male Zucker rats. Shown is wound area development on days 8 and 9 (% reduction of wound size). Wound size was significantly reduced when treated with hu STAT1 (25 µg/wound). In contrast, recombinant PDGF exerted only minor effects as compared to untreated wounds and was profoundly weaker than hu Stat1.

### Examples

### Example 1: Materials & Methods

### 1.1 Cell culture:

Primary cells (SNF, PS) are fibroblasts derived from human skin from patients undergoing plastic surgery. Tissue specimens were cut into small pieces, freed from fat tissue and incubated at 37°C in the presence of collagenase/dispase for 4hrs. Cell lines: AB4 cells are human cells resembling plasmacytoid dendritic cells and are derived from a patient with histiocytosis. ECV304 and A431 are human cell lines of endothelial and epithelial origin, respectively. Cells were cultured in RPMI1640 supplemented with 10% heat-inactivated FCS, 2 mM L-Glutamine and 100 IU/ml Penicillin-100 µg/ml Streptomycin (all reagents from PAA Laboratories, Linz, Austria). Cells were sub-cultured twice weekly at a ratio of 1:4.

### 1.2 Reagents:

Recombinant IL-2, VEGF and PDGF were obtained from PeproTech (London, UK). Bovine Collagen type I and Fibronectin were purchased from Sigma-Aldrich (St. Louis, MO). Hydrogel refers to Normlgel® (Mölnlycke, Gothenburg, Sweden) and the PDGF-containing Hydrogel was obtained through Janssen-Cilag (Regranex®, Belgium).

### 1.3 Cloning, Expression and functional features of human Stathmin1

- Origin: cDNA derived from a human cell linie AB4
- plasmacytoid-like dendritic B-cells as described in patent AT 412281 B.
- Expression in *E. coli* (strain BL21)
- Purification to homogeneity
- Technical details are described in references 1-5.

### 1.3.1 Cloning

AB4 cDNA was generated with ImProm-II Reverse Transcription System (Promega) according to the supplier's instruction. A PCR reaction was set up with Phusion High Fidelity DNA Polymerase, Oligonucleotides "hu STAT1-NcoI-F" and "hu STAT1-Bsu-R" and AB4-cDNA as template to generate a PCR product with NcoI restriction site at the 5'-end and Bsu36I restriction site at the 3'-end.

The PCR product and pTriEx4-Neo plasmid DNA were digested with NcoI and Bsu36I at 37°C for 2 hours. Digestion products were separated by agarose gel electrophoresis. The respective bands for the digested PCR product and plasmid DNA were purified from agarose gel and ligated with T4 DNA Ligase for 4 hours at room temperature. Ligation reaction was transformed into TOP10 One Shot Chemically Competent cells with heat shock at 42°C for 30 sec. Transformation reaction was plated out on LB Agar plates with Ampicillin and incubated for 12 hours at 37°C. Colony PCR was carried out with GoTaq DNA Polymerase and Oligonucleotides "hu STAT1-NcoI-F" and "hu STAT1-Bsu-R" to identify positive transformants.

PCR products were checked on an agarose gel, positive transformants showed a specific band at 450 bp in agarose gel. The positive clones were inoculated into 4 ml LB-Broth with Ampicillin, incubated 12 hours at 37°C.

Plasmid preparation was carried out with High Pure Plasmid Isolation Kit according to supplier's instruction. Plasmid DNA was digested with NcoI and Bsu36I at 37°C for 2 hours to further verify the positive transformants.

hu STAT1 was successfully cloned in pTriEx4-Neo and TOP10 One Shot Chemically Competent cells. After the NcoI/Bsu36I control digestion hu STAT1 in pTriEx4-Neo /TOP10 clones 4, 6 and 8 showed the respective band at 450bp in agarose gel (see Fig.1). Plasmid DNA of the positive transformants was sent to MWG Biotech (Munich, Germany) for sequence verification and evaluation was performed by aligning the resulting sequence with reference hu STAT1 sequence from genbank employing multiple sequence alignment tool.

### 1.3.2 Expression

BL21 DE3 *E. coli* clone 4-3 containing hu Stat1 cloned in pTriEX-4 Neo at NcoI/Bsu36I was grown in LB-Medium containing 100 µg/ml Ampicillin to OD₆₀₀ >0.5. The specific protein band for hu Stat1 (17,3 kD) was induced after addition of 1 mM IPTG. Bacterial cells were harvested by centrifugation at 4,500 rpm for 1 hour. Cell pellet was resuspended in lysis buffer (Pellet from 10 ml culture in 500 µl Lysis Buffer) and exhibited to 3 freeze/thaw cycles to perform cell lysis. Then the lysate was separated into cell debris and supernatant by centrifugation at 4500 rpm for 1 hour. Lysate-supernatant was filtrated through 0.45 µm syringe filter.

### 1.3.3 Anion Exchange Chromatography

The lysate-supernatant was applied to Capto DEAE Sepharose Fast Flow (GE Healthcare, Germany) with Anion Exchange Buffer A on an ÄKTA Purifier FPLC System. Unbound sample was washed from the column with 10CV of Anion Exchange Buffer A, then the column was washed with 10CV of 5% Anion Exchange Buffer B. For elution 5CV of 20% Anion Exchange Buffer B were applied to the column. The eluate was collected in fractions of 15 ml and analyzed on 12% BisTris Gel with Coomassie Stain.

### 1.3.4 Ammoniumsulfate precipitation

The hu STAT1 containing fractions from the Anion Exchange Chromatography were pooled and precipitated with 30% Ammoniumsulfate stirring for 24 hours at 4°C. After precipitation the sample was separated in pellet and supernatant by centrifugation at 4500 rpm for 10 minutes. The supernatant was filtered through 0.45 µm syringe filter.

### 1.3.5 Hydrophobic Interaction Chromatography

The supernatant from Ammoniumsulfate precipitation was applied to Octyl Sepharose Fast Flow with HIC Buffer A1 on an ÄKTA Purifier FPLC System (GE Healthcare). Unbound sample was washed from the column with 10CV of HIC Buffer A. For elution a gradient from 0-100% HIC Buffer B over 20CV was performed. The eluate was collected in fractions of 10 ml and analyzed on 12% BisTris Gel with Coomassie Stain, Silver Stain and Western Blot.

The fractions containing hu STAT1 from the HIC step were pooled and concentrated with a Centrifugal Filter Device (Millipore, Vienna), in this step also buffer was exchanged to 20 mM Tris pH 7.3. Finally the product was sterilized through 0.2 µm syringe filter. The purity of the resulting product was proven by Silver Staining (see Figure 3 lane 10). Protein identity was verified in a target protein specific Western Blot (see Figure 3, lane 12).

The insert sequence of hu STAT1 in pTriEx4 Neo/TOP10 clone 4 is identical with huSTAT1 and correctly ligated into the pTriEx4-Neo vector at the restriction sites 5'-NcoI and 3'-Bsu36I, thus enabling expression of the native protein without vector-encodes amino acids (see Figure 2).

### SDS-PAGE

### 1.3.6 Western Blot analysis of cell lysates and culture supernatant

AB4 and PS-F cells were cultivated in 75 cm² flasks (Nunc) with RPMI, 10% FCS (PAA), then the cultures were kept in serum-free RPMI (PAA) for 48h. After that AB4 cells were harvested by centrifugation and resuspended in 0.5% (v/v) Triton at a concentration of 2x10^6 cells/ml, PS-F cells were treated with Trypsin (PAA) and resuspended in 0.5% Triton at a concentration of 2x10^6. Culture supernatant of both cell lines was collected and concentrated with U-Tube Concentrator, 2H-2 (Merck). Cell lysates and supernatants were separated on a 12% Criterion XT Bis-Tris Gel (Biorad, Vienna) and transferred to PVDF membrane (Carl Roth, Germany) by semi-dry blotting. The membrane was blocked with 3% (w/v) non-fat dry milk powder in TBST for 1h at 37°C followed by three 10min-washes (2x with TBST, 1x with TBS). Serum from a rabbit previously immunized with Stathmin1 was diluted 1:100 in TBST, applied to the membrane and incubated for 24h at 4°C. After three wash steps, the membrane was incubated with Immun-Star Goat Anti-Rabbit (GAR)-HRP Detection Kit (Bio-Rad Laboratories) and analyzed with a ChemiDoc MP system (Bio-Rad Laboratories).

### 1.4 Electric cell impedance sensing

ECIS electrode arrays (8W1E) were obtained from IBIDI, Munich. Before seeding cells, arrays were coated with Fibronectin (Sigma-Aldrich, F1141) at 5 µg/ml in ultra pure water at 37°C for 3 - 4 hours. After coating, aspirate fibronectin, dry at room temperature and stored under sterile conditions. Depending on cell type and application, different cell concentrations may be used; 1 × 10⁵ cells/ml resulted in a confluent layer within 2-3 days

After FN coating, 200µl of serumfree RPMI were added to each well. The arrays were put into the CO₂ incubator for the medium to adjust to the atmosphere to facilitate better cell attachment. Cell proliferation, wounding and cytotoxicity was measured using the ECIS Model 1600R (Applied Biophysics, USA) essentially as described by Keese et al. (8).

### 1.5 Angiogenesis assay

Angiogenesis was measured using ECV304 cells grown in culture vessels coated with fibrinogen/thrombin. Briefly, 30 µL Fibrinogen Solution was dispensed into wells of a flat-bottomed 96-well plate. The plate was gently shaken to ensure that the fibrinogen solution covers the bottom of the well followed by addition of 20 µL/well thrombin to the 96-well plate. Plates were shaken and placed at 37°C for 15-60 min for polymerization. The proteins to be tested (r huStat1) were diluted in serum-free medium. 100 µL of ECV 304 cells (10⁵ cells/ml) in serum-free medium were added to wells of 96-well plate and then additional 100 µl of the respective r hu Stat1 dilutions. As a positive control hu rVEGF-165 (Peprotech, UK) was used. Cultures were incubated at 37°C, 5% CO₂, and analyzed by light microscopy/digital photography after 24, 48 and 72hrs.

### 1.6 NK-mediated cytotoxicity

Heparinized blood was drawn from healthy volunteers and peripheral blood mononuclear cells were isolated by Ficoll density centrifugation. Subsequently, NK+ cells were isolated by negative selection with magnetic beads according to Milteny's protocol. (NK Cell Isolation Kit, Miltenyi Biotec, Bergisch Gladbach, Germany). The purity of the cell population was determined by flow cytometry. The resulting NK-Fraction was harvested, counted and purity was checked by FACS-analysis.

1 x 10⁵ A431 cells were seeded into 8-well ECIS arrays. After 24 hrs, 1 x 10⁶ NK cells were added to each well (target:effector ratio 1:10). Recombinant hu Stat1 at different concentrations were added; r hu IL2 (50 units/ml; Peprotech, UK) was used as a positive control. The impedance of the target cell line was observed for several days in order to assess NK cell mediated killing. To one well of A431, no NK were added in order to determine the time-point at which A431 die without induction.

### 1.7 Gene expression of SN-F cells stimulated with stathmin

SN-F cells (primary human skin fibroblasts) were cultured in RPMI-1640 medium, supplemented with 10% FCS, 2 mM L-glutamin and Penicillin-Streptomycin. After reaching a confluence of about 80%, cells were trypsinized and seeded on 6-well plates with a cell number of 200 000 cells/well. For stimulation, rhuStat1 at concentrations of 5 µg/ml, 50 ng/ml and 500 pg/ml were added to cultures. After the end of stimulation time (2h, 6h, 24h, 48h), cells were detached from the wells and further processed for RNA isolation.

Total RNA was isolated with Promega's SV Total RNA isolation System (Promega, Madison, USA). For that purpose the cells were trypsinised and centrifuged at 800 rpm for 10 minutes. After that the pellet was diluted in 1xPBS and centrifuged at 300 g for 5 minutes and the remaining cell pellet was lysed in 175 µl lysis buffer. Finally 350 µl dilution buffer were added to the lysed cells and the sample was incubated at 70°C for 3 minutes. Then the sample was centrifuged at 13 000 x g for 10 minutes. All further steps were performed according to the manufacturer's protocol.

The resulting RNA preparations were further processed for cDNA synthesis with Promega's "ImProm II™ Reverse Transcription System" (Promega, Madison, USA). For the PCR reaction of the housekeeping gene and the specific genes, two different mastermixes were used. Each mastermix was supplemented with Combinatorial Enhancer Solution (CES), which consists of 2.7 M betaine, 6.7 mM DTT (dithiothreitol), 6.7% DMSO (dimethylsulfoxide) und 55 µg/ml BSA (bovine serum albumine).

Primers were designed with the help of the internet tool Primer Design Assistant (PDA) and synthesized by VBC Oligotech (Vienna, Austria). All primers were used at a final concentration of 10 pmol. Primers are given in table 1:

**Table 1: Primer sequences (SEQ ID NO 4 to 21 in descending order)**

| Growth factor | Primer sequence | Size of product (bp) |
|---|---|---|
| IFN alpha | F: CCTGGATAACAGGAGGACCTTG | 400 |
| | R: CCAGGCACAAGGGCTGTATTTC | |
| IFN beta | F: CTGCCTCAAGGACAGGATGAAC | 350 |
| | R: TGACTATGGTCCAGGCACAGTG | |
| PDGF-B | F: CCCCACACTCCACTCTGATT | 181 |
| | R: GCCCTGGCCTCTAGTCTTCT | |
| FGF-2 | F: ATGAAGGAAGATGGAAGATT | 216 |
| | R: TCAGCTCTTAGCAGACATTG | |
| HGF | F: CAAATGTCAGCCCTGGAGTTCC | 400 |
| | R: AATTGCACAGTACTCCCAGCGG | |
| TGF beta | F: CACGTGGAGCTGTACCAGAA | 239 |
| | R: GAACCCGTTGATGTCCACTT | |
| VEGF-A | F: TCGGGCCTCCGAAACCATGAAC | 320 |
| | R: TGGCCTTGGTGAGGTTTGATCC | |
| KGF | F: ACACCCGGAGCACTACAC | 168 |
| | R: GGGCTGGAACAGTTCACATT | |
| Ribosomal Protein 1 | F: GTCAACATTGGGAGCCTCAT | 176 |
| | R: AGACCAAAGCCCATGTCATC | |

The appropriate number of cycles was determined by comparing 30, 35 and 40 cycles. Given that the best results could be observed after 35 cycles, this number was established for further use.

Agarose gel electrophoresis of the PCR samples was performed in a 1% agarose gel (1 g agarose + 100 ml 1 x Tris borate EDTA (TBE) buffer). Instead of ethidium bromide, the gels were stained with 7 µl Lonza® Gel Star Nucleic Acid stain. For analysis 15 µl of the PCR sample and 10 µl of the DNA ladder (Quick-Load® 100 bp DNA ladder, New England Biolabs) were loaded onto the gel and electrophoresis was run at 115 V for approximately 45 minutes. Pictures of the ready gel was taken under a UV transilluminator.

Image analysis was performed with the software LabImage 1D by Kapelan (Leipig, Germany). With the help of this program the amount of DNA was quantified by comparing the DNA bands of the samples with the DNA amount in the marker.

### 1.8 Human Soluble Receptor Array

Human primary fibroblasts (SN-F) were cultured overnight in the presence of 50 ng/ml TMBP3 (RPMI medium + 10% FCS + TMBP3) or unstimulated (RPMI medium + 10% FCS). After cultivation cells were detached, rinsed with PBS and solubilised in Lysis Buffer (part of array Kit) at a concentration of 1x10⁷ cells/ml. The lysates were rocked at 4°C for 30 minutes. After microcentrifugation for 5 minutes, the supernatant was transferred into a clean test tube. All further steps were done according to R&D's protocol provided with the Proteome ProfilerArray (R&D, Cat. No. ARY012) specific for hu soluble receptors.

After adding the chemiluminescent reagents, the light intensity of the signals was measured with the UVP BioSpectrum AC Imaging System (UVP, Upland, CA.) with an exposure time of 30 minutes. The resulting pictures were analysed with the software Phoretix Array, which is part of the TotalLab TL100 program (Nonlinear Dynamics Newcastle, England). This software calculates the pixel value (light intensity) of each individual spots.

### 1.9 Positron Emission Tomography (PET) studies

Stat1 was labeled with iodine-124 and Chloramine T followed by paper electrophoresis and HPLC for quality control. 3 male Spraque Dawley rats were wounded. Rats were anesthetized and shaved on the back of the body. Two full thickness wounds were prepared by the veterinary surgeon using a biopsy-punch (Fa. Henry Schein ^{®}, diameter: 0.8 cm). After wound setting, animals were positioned on the microPET bed which was kept at 38°C. Animals were treated with 5 µg or 100 µg r hu Stat1 in 50µl hydrogel (Normlgel®, Mölnycke). 4 scans at 0, 4, 8 and 24 hrs were performed using a Focus220 microPET scanner (Siemens Medical Solutions, Munich). Measurements were performed directly after 4, 8, and 24 hrs after radiotracer administration. To cover the whole body of the rat, 10 min static scans at 3 bed positions were acquired. All images were reconstructed using FORE rebinning followed by filtered back projection algorithm. Regions of interest (ROIs) were manually drawn on the reconstructed images.

### 1.10 In vivo wound healing in Zucker rats

Male Zucker rats (Crl:2UC-Lepr ^{fa} (fa/fa) were used as an in vivo model to test the efficacy of r hu Stat1 in delayed wound healing. A single wound was set by a biopsy punch on the shaved back of each rat. Wounds were treated with different concentrations (25.5 µg or 0.5 µg) of r hu Stat1 in 50 µl hydrogel, 50 µl Regranex® or left untreated. Wounds were closed with Varihesive® extra mince (CovaTec) and fixed with Leukoplast. On each animal one circular para-vertebral dermo-epidermic full-thickness wound was set on the back and treated daily topically with either different dosing of hu STAT1 (0.5 µg/wound, 5 µg/wound and 25 µg/wound) or with Regranex® (5 µg/wound). As an additional control one circular para-vertebral dermo-epidermic full-thickness wound was set on non-treated animals. The test item hu STAT1 and the reference item Regranex® were very well tolerated. Rats were treated daily over a period of 12 days. Wounds were evaluated by digital photography which were used for wound size determination. The extent of the individual wound area was expressed as a percentage of the original wound size. The analysis of the healing progress was performed by calculation of the wound area from digital pictures. The effect of the treatments was analyzed using a Mann-Whitney U-test.

### 1.11 Stathmin stability in wound fluid

Purified recombinant Stathmin was diluted in 0,9% (w/v) NaCl, respectively wound fluid and human serum at a concentration of 100µg/ml and incubated at 37°C for a total of 72h. Samples (15µl) were taken at different time intervals and separated on a 12% Criterion XT Bis-Tris Gel (Biorad). Membrane transfer and blocking was performed as described before. Whole Serum from a rabbit previously immunized with Stathmin was diluted 1:1000 in TBST, applied to the membrane and incubated for 1h at room temperature. Following three wash steps (as described before) the membrane was incubated with Goat polyclonal secondary antibody to Rabbit IgG-H&L-AP (Abcam) diluted 1:1000 in TBST for 1h at room temperature. After another three wash steps membrane was covered with NBT/BCIP stock solution for blot & immunhistochemistry (Roche) diluted according to the supplier's guidelines and incubated at room temperature for 10 min.

### 1.12 Hydrogel preparation

Alginate-Gel without propylene glycol:

| | |
|---|---|
| *Alginate-Gel 4%* | *100.0 g* |
| Alginic acid, sodium salt | 4.0 g |
| Demineralised water | 86.0 g |
| *Alginate-Gel 6%* | *100.0 g* |
| Alginic acid, sodium salt | 6.0 g |
| Demineralised water | 84.0 g |

Alginate-Gel with propylene glycol:

| | |
|---|---|
| *Alginate-Gel 4%* | *100.0 g* |
| Alginic acid, sodium salt | 4.0 g |
| Propylene glycol | 5.0 g |
| Demineralised water | 81.0 g |
| *Alginate-Gel 6%* | *100.0 g* |
| Alginic acid, sodium salt | 6.0 g |
| Propylene glycol | 5.0 g |
| Demineralised water | 79.0 g |

A plastic mortar with a pestle has to be tared on an analytic balance. The alginic acid sodium salt has to be weighted into the plastic mortar and an equal amount of demineralized water has to be added. Carefully, the salt will be grinded with this fraction of water. It has to be paid heed to the fact, that the alginic acid sodium salt must not be stuck on the ground of the plastic mortar. To remove it a spatula can be used. Under carefully stirring the remaining water has to be added slowly and partly to the mixture. It has to be paid heed to avoid bubbles in the formulation. After stirring for a while the alginic acid sodium salt will be dispersed in the water and will starting gelling. Now the plastic mortar has to be stored in the refrigerator for at least 12 hours.

The 10 g which are missing at the moment will be added later with the therapeutic protein.

If it is necessary the pH can be adjusted with a NaCl solution

The produced gels have to be sterilized. For this step the samples will be placed in an autoclave for 30 min at 120 °C.

For the addition of stathmin as therapeutic protein, the next steps have to be done under a laminar airflow to ensure antiseptic conditions. The therapeutic protein has to be solved in water. The drug concentration was decided on 500 µg/ml. For 100 g formulation 10 g of a protein solution with the decided concentration has to be filtered through a sterile filter with a pore size of 0.45 µm. The sterile aqueous solution was injected into the gel. Homogenization was thereafter done by mixing the gel and the protein solution in the vessel in which the gel was sterilized. Each formulation had to be homogenized on the vortex. The formulation was then stored in the refrigerator at 2 °C to 8 °C.

### 2. Results

### 2.1 Stathmin is secreted by human cells resembling plasmacytoid dendritic cells

AB4 cells are permanently growing human cells isolated from a lymph node of a patient with histiocytosis. These cells resemble plasmacytoid dendritic cells since they express various functional and phenotypical features of this particular cell type (i.e. membrane antigen expression, antigen presenting capacity, dendritic shape, high IFN-type 1 expression). AB4 cells were cultured in serum-free medium for 48 hrs and both cell lysates and culture supernatants were analyzed by a sensitive western blotting procedure, which could easily detect Stat1 down to a level of 5 ng. In both lysate and supernatants of AB4 cells a clear cut signal at 17,3 kD could be detected by a Stat1-specific rabbit immune serum. In contrast, in primary human skin cells only in cell lysate a faint band could be detected. Culture supernatans were essentially devoid of secreted Stat1 (see Fig.1). Secretion of Stat1 is a remarkable finding since so far this protein has been described in the literature as being associated with cytoskeleton structures and/or TLR3 and is exclusively expressed intracellularly.

### 2.2 Proliferation of human epithelial cells by hu Stat1

Electric cell-substrate impedance sensing was used to carry out wound healing and cell proliferation in tissue culture. The human epithelial cell line A431 was cultured in ECIS electrode arrays for 48hrs. At that time cells have reached confluency and impedance reached a plateau. Data acquisition was briefly suspended, and wells received an elevated field pulse, one for 10 sec and the other for 30 sec; data acquisition was then restarted. Immediately after the pulse, various concentrations of hu Stat1 were added to individual cultures. 10% FCS was added in positive control wells, while negative control cultures received serum-free medium only. Stathmin1 rapidly induced proliferation of cells in the area covering the electrode in a dose-dependent manner, which was comparable to the positive control cultures. Serum-free medium had no effect (Fig 4.).

It is interesting to note, that there was a combined action of Stat1 and collagen type1. While collagen alone had no effect on wound healing in Zucker rats, the combination of Stat1 and collagen nicely restored the wounded area (Fig. 5).

### 2.3 Induction of neoangiogenesis/sprouting/tube-like structures by hu Stat1

Loss of microvessels in the lower limb/foot area and concomitant malnutrition of tissue is one of the hallmarks of DFU. Therefore, it is of utmost importance to reconstitute the micro-vasculature. As can be seen in Fig. 6 the addition of r hu Stat1 to human endothelial cells resulted in a strong response. This was evident by the formation of numerous sprouts, lumina and tube-like structures. In the absence of Stat1, no such reactions were observed. There were also some sprouts/lumina in PDGF-treated cultures but to a much lesser extent.

### 2.4 Stimulation of NK-cell-mediated cytotoxicity by hu Stat1

Almost all chronic wounds are colonized by pathogens and require treatment with antibiotics. Since the wound area is an ideal environment for bacterial growth, a first line defence by either dendritic cells or Natural killer cells would be desirable. There have been reports in the literature, that Stat1 is an agonist for TLR3 (WO 07/089151). However, there was prior to the invention no functional proof that Stat plays a role in immune defence mechanisms.

Therefore an ECIS-based NK assay was designed in which the killing of target cells could be measured by highly enriched human NK cells in real time. The addition of r hu Stat1 indeed led to an activation of NK cells and entire destruction of target cells after less than four days (Fig. 7).

### 2.5 Stat1-mediated Induction of Cytokine/growth factor-specific mRNA in primary human skin fibroblasts

An important feature of chronic wounds is the absence or downregulation of certain cytokine/growth factor gene expression. This is a major drawback, as these proteins support not only epidermal/dermal cell proliferation and migration but also support functions of dendritic cells, mesenchymal stem cells and tissue remodeling. Results are summarized in table 2. As can be seen, primary human skin fibroblasts could be activated by Stat1 to upregulate cytokines/growth factors that are absent in chronic wounds with the exception of PDGF. Furthermore, gene expression of the genes analyzed are vital for cellular functions of immune cells and stem cells residing in the skin.

**Table 2: Activation of growth factor/cytokine genes**

| **Probe** | **assay design** | **DNA (ng)** |
|---|---|---|
| **IFN alpha** | AB4 non-stimulated | 13,3 |
| | SN-F non-stimulated | 0 |
| | SN-F + 500 µg/ml Stathmin 2 Hours | 4,1 |
| | SN-F + 50 ng/ml Stathmin 2 Hours | 0 |
| | SN-F + 5 µg/ml Stathmin 2 Hours | 7,8 |
| | SN-F + 500 pg/ml Stathmin 6 Hours | 3,5 |
| | SN-F + 50 ng/ml Stathmin 6 Hours | 0 |
| | SN-F + 5 µg/ml Stathmin 6 Hours | 38,5 |

| **IFN beta** | RB IgG non-stimulated | 352 |
|---|---|---|
| | SN-F non-stimulated | 0 |
| | SN-F + 500 pg/ml Stathmin 2 Hours | 1,6 |
| | SN-F + 50 ng/ml Stathmin 2 Hours | 0,2 |
| | SN-F + 5 µg/ml Stathmin 2 Hours | 6,5 |
| | SN-F + 500 pg/ml Stathmin 6 Hours | 4,3 |
| | SN-F + 50 ng/ml Stathmin 6 Hours | 5,5 |
| | SN-F + 5 µg/ml Stathmin 6 Hours | 31,6 |
| | SN-F + 500 pg/ml Stathmin ÜN | 38,9 |
| | SN-F + 50 ng/ml Stathmin 6 ÜN | 291 |
| | | |

| **PDGF-B** | AB4 non-stimulated | 116,6 |
|---|---|---|
| | SN-F non-stimulated | 52,1 |
| | SN-F + 500 pg/ml Stathmin 2 Hours | 80,3 |
| | SN-F + 50 ng/ml Stathmin 2 Hours | 73,5 |
| | SN-F + 5 µg/ml Stathmin 2 Hours | 58,9 |
| | SN-F + 500 pg/ml Stathmin 6 Hours | 57 |
| | SN-F + 50 ng/ml Stathmin 6 Hours | 43 |
| | SN-F + 5 µg/ml Stathmin 6 Hours | 55 |
| | SN-F + 500 pg/ml Stathmin 24 Hours | 37,4 |
| | SN-F + 50 ng/ml Stathmin 24 Hours | 30,8 |
| | SN-F + 5 µg/ml Stathmin 24 Hours | 45,5 |
| | SN-F + 500 pg/ml Stathmin 48 Hours | 17,5 |
| | SN-F + 50 ng/ml Stathmin 48 Hours | 42,2 |
| | SN-F + 5 µg/ml Stathmin 48 Hours | 63,1 |
| | | |

| **FGF-2** | AB4 non-stimulated | 81,3 |
|---|---|---|
| | SN-F non-stimulated | 0 |
| | SN-F + 500 pg/ml Stathmin 2 Hours | 267,9 |
| | SN-F + 50 ng/ml Stathmin 2 Hours | 235,5 |
| | SN-F + 5 µg/ml Stathmin 2 Hours | 207,6 |
| | SN-F + 500 pg/ml Stathmin 6 Hours | 51,4 |
| | SN-F + 50 ng/ml Stathmin 6 Hours | 72,3 |
| | SN-F + 5 µg/ml Stathmin 6 Hours | 177,1 |
| | SN-F + 500 pg/ml Stathmin 24 Hours | 236,3 |
| | SN-F + 50 ng/ml Stathmin 24 Hours | 190,3 |
| | SN-F + 5 µg/ml Stathmin 24 Hours | 245,2 |
| | SN-F + 500 pg/ml Stathmin 48 Hours | 187,9 |
| | SN-F + 50 ng/ml Stathmin 48 Hours | 225,2 |
| | SN-F + 5 µg/ml Stathmin 48 Hours | 227,2 |
| | | |

| **HGF** | AB4 non-stimulated | 82,7 |
|---|---|---|
| | SN-F + 5 µg/ml Stathmin, 48 Hours | 31 |
| | | |

| **TGF beta** | SN-F + 500 pg/ml Stathmin 2 Hours | 252 |
|---|---|---|
| | SN-F + 50 ng/ml Stathmin 2 Hours | 222 |
| | SN-F + 5 µg/ml Stathmin 2 Hours | 200 |
| | SN-F + 500 pg/ml Stathmin 6 Hours | 78 |
| | SN-F + 50 ng/ml Stathmin 6 Hours | 108 |
| | SN-F + 5 µg/ml Stathmin 6 Hours | 165 |
| | SN-F + 500 pg/ml Stathmin ON | 30 |
| | SN-F + 50 ng/ml Stathmin ON | 161 |
| | SN-F + 5 µg/ml Stathmin ON | 6,7 |
| | SN-F non-stimulated | 3,1 |
| | AB4 non-stimulated | 130 |
| | SN-F + 500 pg/ml Stathmin 24 Hours | 171 |
| | SN-F + 50 ng/ml Stathmin 24 Hours | 51 |
| | SN-F + 5 µg/ml Stathmin 24 Hours | 166 |
| | SN-F + 500 pg/ml Stathmin 48 Hours | 51 |
| | SN-F + 50 ng/ml Stathmin 48 Hours | 129 |
| | SN-F + 5 µg/ml Stathmin 48 Hours | 118 |
| | | |

| **VEGF-A** | AB4 non-stimulated | 114,6 |
|---|---|---|
| | SN-F non-stimulated | 0 |
| | RB IgG non-stimulated | 0 |
| | SN-F + 500 pg/ml Stathmin 2 Hours | 436 |
| | SN-F + 50 ng/ml Stathmin 2 Hours | 311 |
| | SN-F + 5 µg/ml Stathmin 2 Hours | 347 |
| | SN-F + 500 pg/ml Stathmin 6 Hours | 34 |
| | SN-F + 50 ng/ml Stathmin 6 Hours | 51 |
| | SN-F + 5 µg/ml Stathmin 6 Hours | 246 |
| | SN-F + 500 pg/ml Stathmin 24 Hours | 123 |
| | SN-F + 50 ng/ml Stathmin 24 Hours | 0 |
| | SN-F + 5 µg/ml Stathmin 24 Hours | 119 |
| | SN-F + 500 pg/ml Stathmin 48 Hours | 45 |
| | SN-F + 50 ng/ml Stathmin 48 Hours | 98 |
| | SN-F + 5 µg/ml Stathmin 48 Hours | 100 |
| | | |

| **Ribosomal Protein** | AB4 non-stimulated | 408,1 |
|---|---|---|
| | SN-F non-stimulated | 539,2 |
| | SN-F + 500 pg/ml Stathmin 2 Hours | 494,9 |
| | SN-F + 50 ng/ml Stathmin 2 Hours | 475,1 |
| | SN-F + 5 µg/ml Stathmin 2 Hours | 464,2 |
| | SN-F + 500 pg/ml Stathmin 6 Hours | 411,9 |
| | SN-F + 50 ng/ml Stathmin 6 Hours | 405,4 |
| | SN-F + 5 µg/ml Stathmin 6 Hours | 434 |
| | SN-F + 500 pg/ml Stathmin 24 Hours | 466,5 |
| | SN-F + 50 ng/ml Stathmin 24 Hours | 377,4 |
| | SN-F + 5 µg/ml Stathmin 24 Hours | 426,7 |
| | SN-F + 500 pg/ml Stathmin 48 Hours | 337,6 |
| | SN-F + 50 ng/ml Stathmin 48 Hours | 322,5 |
| | SN-F + 5 µg/ml Stathmin 48 Hours | 348,5 |
| | | |

| **KGF** | AB4 non-stimulated | 0 |
|---|---|---|
| | SN-F non-stimulated | 169 |
| | SN-F + 500 pg/ml Stathmin 2 Hours | 155,1 |
| | SN-F + 50 ng/ml Stathmin 2 Hours | 113,8 |
| | SN-F + 5 µq/ml Stathmin 2 Hours | 102,8 |
| | SN-F + 500 pg/ml Stathmin 6 Hours | 28,7 |
| | SN-F + 50 ng/ml Stathmin 6 Hours | 33,1 |
| | SN-F + 5 µg/ml Stathmin 6 Hours | 65 |
| | SN-F + 500 pg/ml Stathmin 24 Hours | 194,2 |
| | SN-F + 50 ng/ml Stathmin 24 Hours | 87,6 |
| | SN-F + 5 µg/ml Stathmin 24 Hours | 206,4 |
| | SN-F + 500 pg/ml Stathmin 48 Hours | 145,7 |
| | SN-F + 50 ng/ml Stathmin 48 Hours | 184,8 |
| | SN-F + 5 µg/ml Stathmin 48 Hours | 223 |

### 2.6 Stat1 upregulates IL-8 and represses TIMP-1 and -2 expression

To extend the analysis of proteins stimulated by Stat1 protein array studies have been carried out. In Fig. 8a and 8b results of proteins (IL-8, TIMP-1,-2) important for wound healing are shown. Il-8, which is an important chemoattractant for PMNs is stimulated 8-fold. Bioactive IL-8 is expressed in wounds and enhances wound healing.

Interestingly, Stat1 represses tissue inhibitor of metalloproteinases (TIMPs) a class of enzymes that inhibit metalloproteinases (MMPs) by 50%. MMPs are important for activties such as scar resorption, and re-epithelialization during wound healing. Downregulation of TIMPs may be crucial for remodeling during wound healing.

### 2.7 Stability of rhu Stat1

Proteins become rapidly degraded in wound fluid. A prerequisite for the use of Stat1 as a biologic for treatment would be its stability both in wounds as well as formulated in a hydrogel, the formulation planned to be used in clinical trials. In order to test the stability of the protein, two lines of experiments were conducted:
1. Recombinant Samples were incubated in the presence of wound fluid at 37°C, followed by western blot analysis over the time indicated in Fig. 10. As can be seen, Stat1 was detectable up to 8 hrs, which is enough time to induce cellular functions which are absent in chronic wounds.
2. Recombinant Samples were mixed with a hydrogel (Normlgel, Mölnycke) and incubated at 4°C, -20°C and -80°C. Samples were taken at the times indicated in Fig. 10 and analysed by SDS-PAGE. Results show that the samples remain intact without any signs of degradation for at least 5 month and therefore are perfectly suited for further application in the clinic.

### 2.8 Distribution of hu Statlafter topical application - microPET Imaging

The aim of the study was the evaluation of local and system-ical distribution of radiolabelled hu STAT1 after topical administration into a full thickness wound.

The study showed that the tissue distribution and pharmacokinetics of ¹²⁴I-labelled hu STAT1 can be measured using microPET technology. A difference in radioactivity distribution was found between the 5 µg and 100 µg hu STAT1 in treated animals, starting 4 hours after substance application (Fig. 11a and 11b). Radioactivity levels were also detectable in the bladder, stomach and thyroid. Besides these organs, no uptake of radiolabelled hu STAT1 was observed in any compartment of the body. The higher levels in stomach, thyroid and blood might be related to uptake of the free iodine, as the ¹²⁴I-hu STAT1 stock solution already contained 4% of free iodine, even though radioactivity uptake in these organs is less than reported in the literature. The radioactivity uptake in bladder of all test subjects indicates renal elimination of radiolabelled product.

In conclusion, this study clearly indicates that the vast majority of ¹²⁴I-hu STAT1 remains in the wound area. No other organs were visible in the PET images pointing to no substantial uptake of ¹²⁴I-huStathmin in other areas of the body.

### 2.9 hu STAT1 induces dose-dependent, accelerated wound healing in Zucker rats.

The purpose of this study was to test the efficacy of hu STAT1 in comparison to Regranex® (PDGF) in a wound healing model using male Zucker rats which represent a model for the investigation of chronic wounds. It should be noted that Zucker rats develop a metabolic syndrome and have a delayed wound healing as compared to normal animals. The most relevant wound healing phase in this *in vivo* system starts with day 8.

From study day 8 on, the hu STAT1 treated animals (groups 3, 4 and 5) show biologically significant smaller wound areas compared to the PDGF treated animals (Fig.12). Despite that, the healing of the wounds follows a dose dependent manner. Moreover, hu STAT1 accelerates wound healing. The fastest healing of the wounds was recorded in the hu STAT1 high dose group (25 µg/wound) with statistically significant advantages over the PDGF group on days 9 and 10. Taken together, hu STAT1 accelerates healing in animals with chronic wounds and is superior to current standard treatments.

In combination of stathmin with collagen wound healing was further increased with rapid epithelialization and wound closure (Fig. 5).

### 3. Summary

Stathmin speeds up delayed wound healing in *Zucker* rats and is not systemically active. In addition, it combines several advantages which are crucial for healing of chronic wounds: promotion of skin cell proliferation, induction of angiogenesis and activation of the natural killer cell activity.

Moreover, recombinant Stat1 is useful for activation of specific cytokine genes and proteins important for immune modulation and cancer therapy.

### References

1. Ausubel et al. (1994): Current Protocols in Molecular Biology; in Greene Publishing Associates and Wiley-Interscience, New York.
2. Brennan W. A. and Lin S. H. (1996): Strategies for Protein Purification and Characterization, A Laboratory Manual in Marshak D. K., Burgess J., Knuth R., Brennan M. and Lin S. H., in Cold Spring Harbor Laboratory Press, New York.
3. Dubendorff J. W. and Studier F. W. (1991): Controlling basal expression in an inducible T7 expression system by blocking the target T7 promoter with lac repressor. Journal of Molecular Biology 219, 45-59.
4. Hoskins et al. (1987): Cloning and characterization of human liver cDNA encoding a protein S precursor. Proc. Natl. Acad. Sci. USA 84, 349 353
5. Innis et al. (1990): PCR Protocols: A Guide to Methods and Applications. Academic Press, San Diego, CA.
6. Sambrook J., Fritsch E. F. and Maniatis T. (1989): Molecular Cloning: A Laboratory Manual, Second Edition. Cold Spring Harbor Laboratory Press, New York.
7. Studier F. W. and Moffatt B. A. (1986): Use of bacteriophage T7 RNA polymerase to direct selective high-level expression of cloned genes. Journal for Molecular Biology 189, 113-130.
8. Keese et al. (2004): Electrical wound-healing assay for cells in vitro. PNAS Vol. 101/6: 1555-1559
9. Tan et al. (2007) J Mater Sci Mater Med. 18(10):1961-8.

## Claims

1. Stathmin, a nucleic acid encoding said stathmin or a cell expressing stathmin for use in the treatment of chronic wounds or disease- or medication-dependent impaired wound healing in a patient.

2. Stathmin formulated with or coupled to a biocompatible matrix, preferably comprising collagen, gelatin, chitosan and/or hyaluronan, for use in the treatment of wounds, preferably chronic wounds or diseases- or medication-dependent impaired wound healing, in a patient.

3. Stathmin, a nucleic acid or cell for use according to claim 1 or 2, wherein said stathmin is human stathmin.

4. Stathmin or cell for use according to claim 1, 2 or 3, wherein said stathmin is recombinant stathmin.

5. Stathmin, a nucleic acid or cell for use according to any one of claims 1 to 4, wherein the stathmin comprises amino acids 1 to 126 of SEQ ID NO: 1, or an amino acid sequence that has at least 70 % sequence identity with the sequence of amino acids 1 to 126 of SEQ ID NO: 1.

6. Stathmin for use according to any one of claims 1 to 5, said stathmin being formulated in a hydrogel.

7. Stathmin, a nucleic acid or cell for use according to any one of claims 1 to 6, wherein said patient is in need of a induction of angiogenesis, in need of a stimulation of the innate immune response and/or in need of an induction of cytokines/growth factors and TIMPs for immune modulation.

8. Stathmin, a nucleic acid or cell for use according to any one of claims 1 to 7, wherein said disease-dependent impaired wound healing is due to a disease selected from Diabetes or metablic syndrome, Chronic venous insufficiency (CVI), peripheral artery occlusive disease (PAOD), cancer, autoimmunity, especially an autoimmunity selected from rheumatoid arthritis, lupus and livedoid vasculopathy, ostomy, prolonged inflammatory processes, decubitus, preferably wherein said patient suffers from diabetes or metabolic syndrome.

9. Stathmin, a nucleic acid or cell for use according to any one of claims 1 to 8, wherein said medication-dependent impaired wound healing is due to a medication selected from treatments with a cortiocosteroid, nicotine, an antibiotic, an immunosuppressant, an anti-coagulant, a cytotoxic medication, an anti rheuma-medication, a vasoconstrictor.

10. Stathmin, a nucleic acid or cell for use according to any one of claims 1 to 9, wherein said wound is selected from a chronic or non-healing diabetic foot wound, an ulcer, in particular venous ulcer, a decubitus or pressure ulcer, burns, preferably a third degree burn, a surgical wound, an accidental wound, a necrotic wound, an infected wound.

11. Pharmaceutical composition comprising stathmin, optionally further defined as in any one of claims 3 to 6 formulated with or coupled to a biocompatible matrix, preferably comprising collagen, gelatin, chitosan and/or hyaluronan.

12. Pharmaceutical composition according to claim 11 for use as a medicament.

13. Pharmaceutical composition according to claim 11 for use in the treatment of wounds, preferably as further defined as in any one of claims 1, 7 to 10.

14. In vitro method of increasing or inducing the proliferation and/or migration of mesenchymal cells, epidermal cells and/or stem cells, in particular stromal stem cells, for stimulating immune cells, preferably natural killer cells, for stimulating fibroblasts, for stimulating epithelial cells, preferably epithelial cells of the epidermis, or for stimulating angiogenesis, in particular for stimulating IL-8 production in said cells, especially in mesenchymal cells and/or fibroblasts.

15. The method of increasing or inducing the proliferation and/or migration of mesenchymal cells and/or stem cells, in particular stromal stem cells, for stimulating immune cells, preferably natural killer cells, for stimulating fibroblasts, for stimulating epithelial cells, preferably epithelial cells of the epidermis, or for stimulating angiogenesis, in particular for stimulating IL-8 production in said cells, especially in mesenchymal cells and/or fibroblasts, in a patient
comprising the step of administering stathmin, a nucleic acid encoding said stathmin or a cell expressing stathmin to the patient, preferably by topical administration on a wound.


SEQUENCE LISTING

<110>  Tissue Med Biosciences

<120>  Medicament for wound treatment

<130>  r52232

<160>  21

<170>  PatentIn version 3.3

<210>  1
<211>  149
<212>  PRT
<213>  Homo sapiens

<400>  1

Met Ala Ser Ser Asp Ile Gln Val Lys Glu Leu Glu Lys Arg Ala Ser
1               5                   10                  15

Gly Gln Ala Phe Glu Leu Ile Leu Ser Pro Arg Ser Lys Glu Ser Val
            20                  25                  30

Pro Glu Phe Pro Leu Ser Pro Pro Lys Lys Lys Asp Leu Ser Leu Glu
        35                  40                  45

Glu Ile Gln Lys Lys Leu Glu Ala Ala Glu Glu Arg Arg Lys Ser His
    50                  55                  60

Glu Ala Glu Val Leu Lys Gln Leu Ala Glu Lys Arg Glu His Glu Lys
65                  70                  75                  80

Glu Val Leu Gln Lys Ala Ile Glu Glu Asn Asn Asn Phe Ser Lys Met
                85                  90                  95

Ala Glu Glu Lys Leu Thr His Lys Met Glu Ala Asn Lys Glu Asn Arg
            100                 105                 110

Glu Ala Gln Met Ala Ala Lys Leu Glu Arg Leu Arg Glu Lys Asp Lys
        115                 120                 125

His Ile Glu Glu Val Arg Lys Asn Lys Glu Ser Lys Asp Pro Ala Asp
    130                 135                 140

Glu Thr Glu Ala Asp
145

<210>  2
<211>  174
<212>  PRT
<213>  Homo sapiens

<400>  2

Met Ala Ser Ser Asp Ile Gln Val Lys Glu Leu Glu Lys Arg Ala Ser
1               5                   10                  15

Gly Gln Ala Phe Glu Leu Ile Leu Ser Pro Arg Ser Lys Glu Ser Val
            20                  25                  30

Pro Glu Phe Pro Leu Ser Pro Pro Lys Lys Lys Asp Leu Ser Leu Glu
        35                  40                  45

Glu Ile Gln Lys Lys Leu Glu Ala Ala Glu Glu Arg Arg Lys Ser His
    50                  55                  60

Glu Ala Glu Val Leu Lys Gln Leu Ala Glu Lys Arg Glu His Glu Lys
65                  70                  75                  80

Glu Val Leu Gln Lys Ala Ile Glu Glu Asn Asn Asn Phe Ser Lys Met
                85                  90                  95

Ala Glu Glu Lys Leu Thr His Lys Met Glu Ala Asn Lys Glu Asn Arg
            100                 105                 110

Glu Ala Gln Met Ala Ala Lys Leu Glu Arg Leu Arg Glu Lys Met Tyr
        115                 120                 125

Phe Trp Thr His Gly Pro Gly Ala His Pro Ala Gln Ile Ser Ala Glu
    130                 135                 140

Gln Ser Cys Leu His Ser Val Pro Ala Leu Cys Pro Ala Leu Gly Leu
145                 150                 155                 160

Gln Ser Ala Leu Ile Thr Trp Ser Asp Leu Ser His His His
                165                 170

<210>  3
<211>  450
<212>  DNA
<213>  Homo sapiens

<400>  3
atggcttctt ctgatatcca ggtgaaagaa ctggagaagc gtgcctcagg ccaggctttt      60

gagctgattc tcagccctcg gtcaaaagaa tctgttccag aattccccct ttcccctcca     120

aagaagaagg atctttccct ggaggaaatt cagaagaaat tagaagctgc agaagaaaga     180

cgcaagtccc atgaagctga ggtcttgaag cagctggctg agaaacgaga gcacgagaaa     240

gaagtgcttc agaaggcaat agaagagaac aacaacttca gtaaaatggc agaagagaaa     300

ctgacccaca aaatggaagc taataaagag aaccgagagg cacaaatggc tgccaaactg     360

gaacgtttgc gagagaagga taagcacatt gaagaagtgc ggaagaacaa agaatccaaa     420

gaccctgctg acgagactga agctgactaa                                    450

<210>  4
<211>  22
<212>  DNA
<213>  Artificial

<220>
<223>  primer

<400>  4
cctggataac aggaggacct tg                                              22

<210>  5
<211>  22
<212>  DNA
<213>  Artificial

<220>
<223>  primer

<400>  5
ccaggcacaa gggctgtatt tc                                              22

<210>  6
<211>  22
<212>  DNA
<213>  Artificial

<220>
<223>  primer

<400>  6
ctgcctcaag gacaggatga ac                                              22

<210>  7
<211>  22
<212>  DNA
<213>  Artificial

<220>
<223>  primer

<400>  7
tgactatggt ccaggcacag tg                                              22

<210>  8
<211>  20
<212>  DNA
<213>  Artificial

<220>
<223>  primer

<400>  8
ccccacactc cactctgatt                                                 20

<210>  9
<211>  20

<212>  DNA
<213>  Artificial

<220>
<223>  primer

<400>  9
gccctggcct ctagtcttct                                                   20


<210>  10
<211>  20
<212>  DNA
<213>  Artificial

<220>
<223>  primer

<400>  10
atgaaggaag atggaagatt                                                   20


<210>  11
<211>  20
<212>  DNA
<213>  Artificial

<220>
<223>  primer

<400>  11
tcagctctta gcagacattg                                                   20


<210>  12
<211>  22
<212>  DNA
<213>  Artificial

<220>
<223>  primer

<400>  12
caaatgtcag ccctggagtt cc                                                22


<210>  13
<211>  22
<212>  DNA
<213>  Artificial

<220>
<223>  primer

<400>  13
aattgcacag tactcccagc gg                                                22


<210>  14
<211>  20
<212>  DNA
<213>  Artificial

<220>
<223>  primer

<400>  14
cacgtggagc tgtaccagaa                                                   20


<210>  15
<211>  20
<212>  DNA
<213>  Artificial

<220>
<223>  primer

<400>  15
gaacccgttg atgtccactt                                                   20


<210>  16
<211>  22
<212>  DNA
<213>  Artificial

<220>
<223>  primer

<400>  16
tcgggcctcc gaaaccatga ac                                                22


<210>  17
<211>  22
<212>  DNA
<213>  Artificial

<220>
<223>  primer

<400>  17
tggccttggt gaggtttgat cc                                                22


<210>  18
<211>  18
<212>  DNA
<213>  Artificial

<220>
<223>  primer

<400>  18
acacccggag cactacac                                                     18


<210>  19
<211>  20
<212>  DNA
<213>  Artificial

<220>
<223>  primer

<400>  19
gggctggaac agttcacatt                                                   20

<210>  20
<211>  20
<212>  DNA
<213>  Artificial

<220>
<223>  primer

<400>  20
gtcaacattg ggagcctcat                                                    20


<210>  21
<211>  20
<212>  DNA
<213>  Artificial

<220>
<223>  primer

<400>  21
agaccaaagc ccatgtcatc                                                    20